# EUROPEAN PATENT SPECIFICATION

(11) **EP 4 409 325 B1**
(45) Date of publication and mention of the grant of the patent: **05.08.2026**
(21) Application number: 22785973.3
(22) Date of filing: 13.09.2022
(51) Int. Cl.: G01S 7/52, A61B 8/00, A61B 8/08

(54) **ULTRASONIC DIAGNOSTIC IMAGING SYSTEM WITH TGC CONTROL**
DIAGNOSTISCHES ULTRASCHALL-BILDERZEUGUNGSSYSTEM MIT TGC-STEUERUNG
SYSTÈME D'IMAGERIE DIAGNOSTIQUE ULTRASONORE AVEC COMMANDE DE COMPENSATION DE GAIN TEMPOREL

(30) Priority: 27.09.2021 US 202163248598 P
(43) Date of publication of application: 07.08.2024
(73) Proprietor: Koninklijke Philips N.V., 5656 AE Eindhoven (NL)
(72) Inventor: WIGEN, Jason, 5656 AG Eindhoven (NL); CHANG, Kyong, 5656 AG Eindhoven (NL); FOX, Shannon, Renee, 5656 AG Eindhoven (NL); CHEW, Rita, Kathleen, 5656 AG Eindhoven (NL)
(74) Representative: Philips Intellectual Property & Standards
(86) International application number: PCT/EP2022/075433
(87) International publication number: WO 2023/046536

(56) References cited:
- EP-A1- 3 441 006
- US-A1- 2008 161 688
- US-A1- 2016 081 662

## Description

This invention relates to improvements in ultrasonic diagnostic imaging systems and, in particular, to techniques for controlling signal gain as a function of depth during reception of ultrasonic echo signals.

Ultrasonic imaging systems create images of the interior of a patient's body from echoes received in response to the transmission of ultrasonic waves into the body of the patient. Ultrasonic pulses are transmitted over a field of interest in the body along a plurality of beam direction, causing echoes to return from along each beam direction as the transmitted pulse encounters tissue structures and interfaces in the body. By mapping the received echoes as a function of their time of return and direction an image of the interior of the body can be assembled and displayed.

It is well known that as ultrasonic waves propagate through the body they are continually attenuated and scattered by their passage through the tissue of the body. Echoes are similarly affected during their return. Hence, echoes returning from increasing depths in the body will exhibit ever increasing attenuation. To compensate for this attenuation ultrasonic systems have traditionally amplified returning echoes as a function of depth.

As echoes return from increasing depths, they are processed by increasing amplification. Since the transmitted pulses proceed through the body with time and echoes return from increasing depths at increasing time periods following transmission of each pulse, this amplification is usually controlled by varying the gain of an amplifier in the ultrasound receiver as a function of the time following pulse transmission. This form of gain control is referred to as time gain compensation, or TGC.

It has been customary for an ultrasound system to have a row of gain setting switches which may be set by the user to adjust the TGC. Each switch is an input to the TGC function generator which produces the TGC function over a portion of the reception period following pulse transmission. If there are five switches, for instance, five different variations in gain can be sequentially applied over the reception period during which echoes are received from the shallowest to the deepest depth. The TGC adjustments are generally made to a starting TGC gain characteristic which is provided by the ultrasound system for a particular exam type. The TGC switches, traditionally slide potentiometers with a central reference position, are then used to fine-tune the starting TGC characteristic provided by the ultrasound system.

The sliding switches, or slide pots as they are called, are mechanical components. They are thus subject to wear, deterioration, and mechanical breakdown over time. In addition, the sliders of the slide pots move along slots in the ultrasound system control panel. These slots are openings where dust and other particles can build up, becoming a source of contaminants in an otherwise clean hospital environment. Accordingly it is desirable to provide TGC controls which avoid these shortcomings while still providing a tactile sensation to the sonographer during TGC adjustment.

US 2008/161688 A1 describes an ultrasonic diagnostic imaging system which is operable in a docked mode or in a portable mode. In the docked mode a portable ultrasound system is docked with a docking station.

US 2016/081662 A1 describes systems and methods for gain auto-correction. Automatic gain optimization or correction may be applied in an ultrasound system.

EP 3441006 A1 describes an ultrasound apparatus comprising a communication interface configured to receive an ultrasound image from an external device.

According to an aspect of the invention, there is provided an ultrasonic diagnostic imaging system for producing ultrasound images adjusted for depth-dependent ultrasound attenuation by TGC (time gain compensation) control according to claim 1.

According to another aspect of the invention, there is provided a method for producing ultrasound images adjusted for depth-dependent ultrasound attenuation by TGC (time gain compensation) control according to claim 12.

In the drawings:
FIGURE 1 illustrates an ultrasound display screen with an ultrasound image and TGC characteristic in accordance with the present invention.
FIGURE 2 illustrates an ultrasound system control panel with slide pot TGC control switches.
FIGURE 3 is a close-up view of a set of slide pot TGC controls and the slots for the sliders.
FIGURE 4 illustrates in block diagram form an ultrasound system according to a proposed embodiment.
FIGURE 5 is a plan view of a TGC control module of the present invention.
FIGURE 6 is a side view of a TGC control module of the present invention.
FIGURE 7 illustrates in block diagram form the control signal path for one of the depth zones of a TGC control system of the present invention.
FIGURE 8 illustrates an example method for producing ultrasound images adjusted for depth-dependent ultrasound attenuation by TGC control.

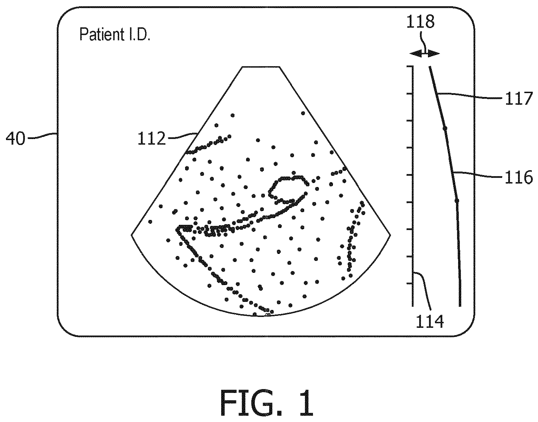

Referring first to FIGURE 1, an ultrasonic image display 40 is shown. In the center of the display is an ultrasound image 112 which shows the tissue structure or flow conditions of the patient being examined. In the upper left corner of the display is alphanumeric information concerning the patient and/or other characteristics of the examination being performed. To the right of the ultrasound image 112 is a depth scale 114 aligned with the image, indicating the depth into the body to which the image extends. Usually the markers on the depth scale are calibrated in centimeters of depth.

To the right of the depth scale 114 is a graphic representation 116 of the TGC characteristic. The TGC characteristic is shown as a sequence of line segments joined by dots on the display. The relative slope of each line segment indicates the variation in gain applied to the received echo signals over the depth covered by that segment. Adjusting an individual TGC switch, as discussed below, will vary the slope of a respective line segment. Each line segment and its switch may have a predetermined, fixed depth over which it is effective, or the segments can be scaled in relation to the maximum depth of the particular image. An initial gain adjustment is used to vary the gain of the entire TGC characteristic, and causes the displayed characteristic 116 to move left or right as indicated by the arrow 118.

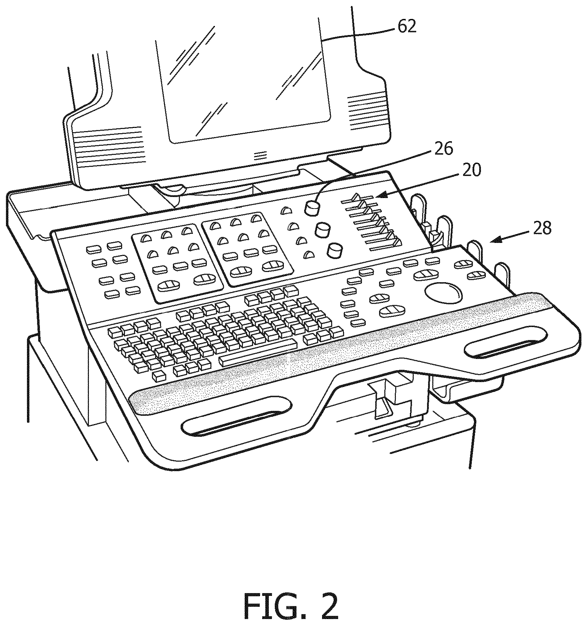

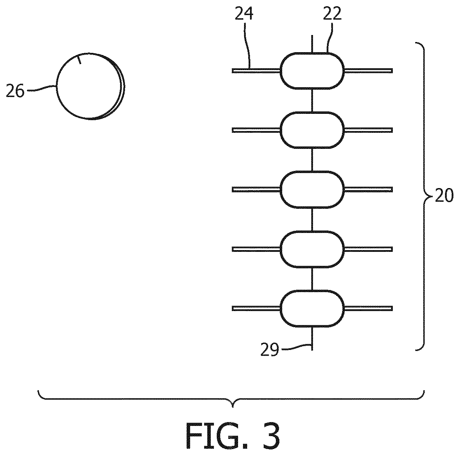

Each segment of the TGC characteristic is set by one of the TGC switches 20 shown on the control panel in FIGURE 2. Conventionally the TGC switches are slide switches such as indicated by the first switch 22 which slides horizontally along the slot 24 in the enlarged view of the TGC control area in FIGURE 3. Switch 22 controls the gain over an initial depth portion of the image as indicated by the first segment 117 of the TGC characteristic 116. Moving slide switch 22 to the right will increase the gain over this initial depth, and will cause the first line segment 117 on the display to slope more steeply to the right. Turning the overall gain control adjustment knob 26 will cause the gain over the full depth to vary, and the TGC characteristic to move to the right or left as indicated by the arrow 118. When all of the TGC switches 20 are vertically aligned along the center line 29 as shown in FIG. 3, there will be no variation in gain over the depth of the image other than that imposed by the starting nominal TGC gain characteristic. If various ones of the TGC switches 20 are progressively moved to the right as shown on the user control console in FIGURE 2, an increasingly sloping TGC characteristic 116 as shown in FIGURE 1 will result.

When a user desires to perform a particular ultrasound examination such as imaging the liver, the user selects the desired procedure by using the controls on the control panel 28. This may involve interaction with a menu of parameters and performance choices shown on the display monitor 62. If the user selects abdominal scanning of the liver with a particular transducer probe, this information is communicated to a system setup controller from the control panel. The setup controller then looks up the control parameters for such a procedure in a setup memory and initializes the system to control the probe and echo signal processing specifically for this procedure. The system beamformer will be set up by the setup controller to activate and receive echo signals from the selected probe, for instance. The setup memory also supplies information to the setup controller as to a nominal TGC characteristic to be used in scanning the liver. The setup controller will then control the gain of the system's TGC amplifiers in accordance with this nominal TGC characteristic. The setup controller will also supply graphical information to the system's graphic processor so that a visual representation of the nominal TGC characteristic will be shown on the image display, as shown as 116 in FIGURE 1.

As the ultrasound exam proceeds, the user may find that the ultrasound image is less than optimal at certain depths. If the user finds that variation from the predetermined TGC characteristic is needed to better image a particular patient, the user will move the slide switches to the right or left to adjust the slope segments of the TGC characteristic. As the switches are moved the changes are communicated from the control panel 28 to a TGC controller, which applies the incremental changes to the predetermined characteristic. The effects of these changes are shown by visual changes to the displayed TGC characteristic 116. When the user is finished adjusting the TGC switches 20 the variation from the predetermined characteristic is indicated by the new physical positions of the switches and the final TGC characteristic is shown on the display. A uniform gain adjustment over the full image depth is applied as before by adjusting the gain control adjustment 26.

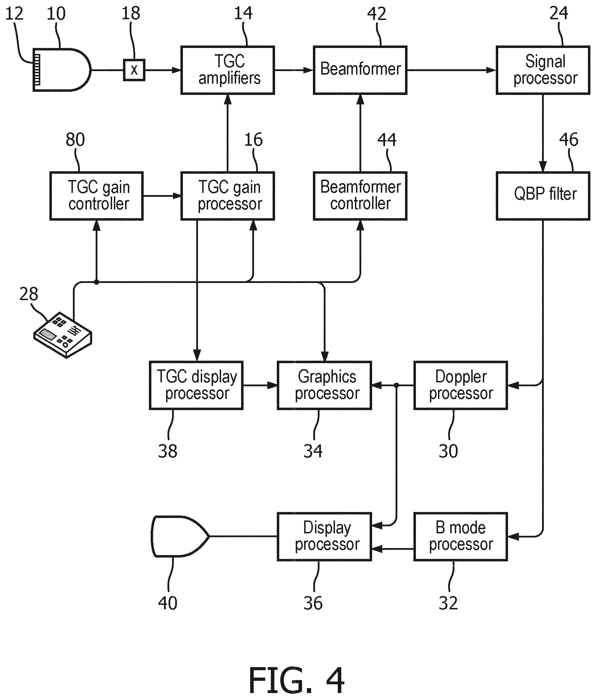

An ultrasound system according to a proposed embodiment is shown in block diagram form in FIGURE 4. A transducer array probe 10 includes an array transducer 12 for transmitting ultrasonic waves and receiving echo signals. The transducer array 12 in this implementation is a one-dimensional (1D) array of transducer elements capable of scanning an azimuth plane in front of the row of elements by steering and focusing beams in the plane. The transducer array may alternatively be a 1.5D (or 1.xD) array transducer with a few elements on either side of a central row for elevation aperture shaping. A 2D array is used for three-dimensional scanning. The elements of the transducer array are coupled by a probe cable to a transmit/receive switch 18 which switches between transmission and reception and protects the beamformer 42 from high energy transmit signals. FIGURE 1 illustrates the major components of the receive signal path of the ultrasound system. The received echo signals are amplified by TGC amplifiers 14 which amplify the signals in a time-gain controlled manner. Generally there is a separate TGC amplifier for each channel of the beamformer to amplify the echo signals from a particular transducer element or group (patch) of elements. The TGC amplifiers are gain-controlled by a control signal provided by a TGC gain processor 16 as described more fully below. The TGC gain processor adjusts the gain in response to gain adjustments made by the user with a TGC gain control module on the control panel 28, as more fully described below. The TGC gain processor also produces gain values for processing by a TGC display processor 38 and display of a TGC characteristic 116 on display 40 by a graphics processor 34 and a display processor 36. TGC gain is initialized by a nominal TGC gain characteristic stored with other setup parameters in a setup memory, which in this illustration is part of setup controller 80, which includes a processor for selecting the proper initial parameters for a desired ultrasound procedure and providing them to other elements of the system.

The amplified echoes received by elements of the array are beamformed by the beamformer 42 by appropriately delaying them and then combining them to produce a coherent echo signal. For example, the beamformer 42 may have 128 channels, each of which controls transmission by and delays signals received from a particular element of a 128-element array transducer. Beamformers may process echo signals in their received analog form or may digitize signal samples and process the echo signals digitally.

The coherent echo signals undergo signal processing by a signal processor 24, which includes filtering by a digital filter and noise (speckle) reduction as by spatial or frequency compounding. The digital filter of the signal processor 24 can be a filter of the type disclosed in U.S. Patent No. 5,833,613 (Averkiou et al.), for example. The echo signals are then coupled to a quadrature bandpass filter (QBP) 46. The QBP filter performs three functions: band limiting the RF echo signal data, producing in-phase and quadrature pairs (I and Q) of echo signal data, and decimating the digital sample rate. The QBP filter comprises two separate filters, one producing in-phase samples (I) and the other producing quadrature samples (Q), with each filter being formed in a digital implementation by a plurality of multiplier-accumulators (MACs) implementing an FIR filter.

The beamformed and processed coherent echo signals are coupled to a pair of image data processors. A B mode processor 32 produces image data for a B mode image of structure in the body such as tissue. The B mode processor performs amplitude (envelope) detection of quadrature demodulated I and Q signal components by calculating the echo signal amplitude in the form of (I²+Q²)^{½}. The quadrature echo signal components are also coupled to a Doppler processor 30. The Doppler processor 30 stores ensembles of echo signals from discrete points in an image field which are then used to estimate the Doppler shift at points in the image with a fast Fourier transform (FFT) processor. The rate at which the ensembles are acquired determines the velocity range of motion that the system can accurately measure and depict in an image. The Doppler shift is proportional to motion at points in the image field, e.g., blood flow and tissue motion. For color Doppler image data, the estimated Doppler flow values at each point in a blood vessel are wall filtered and converted to color values using a look-up table. The wall filter has an adjustable cutoff frequency above or below which motion will be rejected such as the low frequency motion of the wall of a blood vessel when imaging flowing blood. The B mode image data and the Doppler flow values are coupled to a display processor 36 which scan converts the B mode and Doppler samples from their acquired R-θ coordinates to Cartesian (x,y) coordinates for display in a desired display format, e.g., a rectilinear display format or a sector display format. Either the B mode image or the Doppler image may be displayed alone, or the two shown together in anatomical registration in which the color Doppler overlay shows the blood flow in B mode processed tissue and vessels in the image. Color Doppler values may also be coupled to the graphics processor 34 for assembly of a color map of motion or flow to overlay in anatomical registration over a B mode image. Another display possibility is to display side-by-side images of the same anatomy which have been processed differently. This display format is useful when comparing images. The ultrasound images and their associated information is displayed on an image display 40.

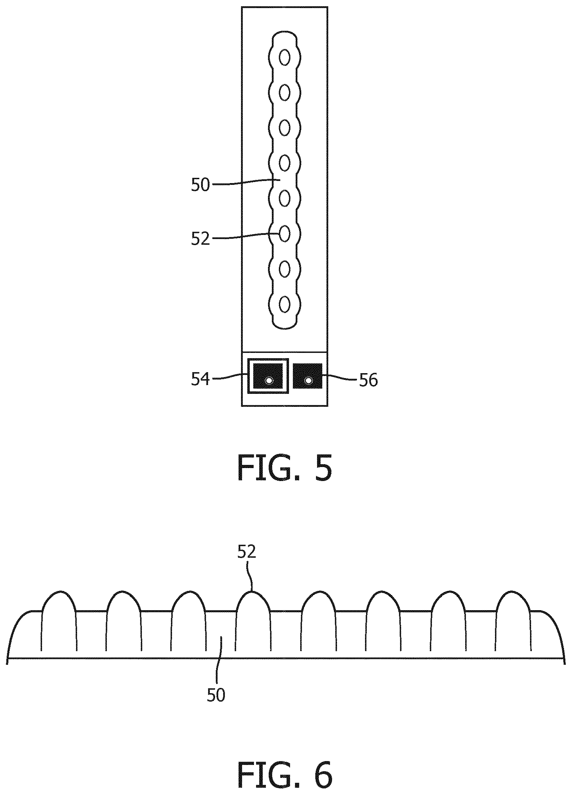

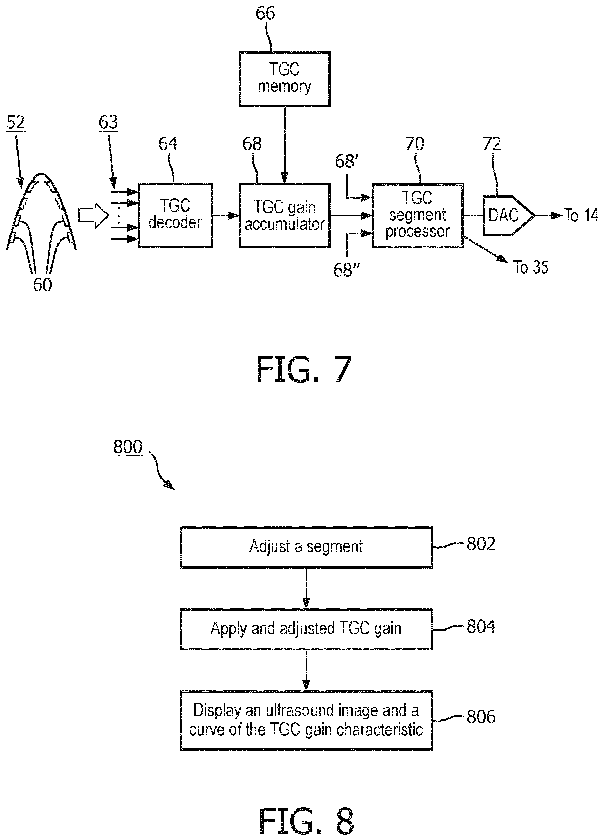

A TGC control module 50 according to a proposed embodiment is shown in a plan view in FIGURE 5. It is also shown in a side view in FIGURE 6. The module is a continuous elongated structure with periodic enlarged regions 52 where the structure is wider and higher than the intermediate areas. A typical module may be 10-15 cm. long and 2-4 cm. high. The module is constructed as a polymeric base of the shape and configuration shown in the drawings. Touch sensors 60, shown in FIGURE 7, are affixed to the lateral sides of the enlarged regions 52. The entire assembly is covered with a smooth polymeric coating such as polystyrene or polycarbonate. Below the TGC control module 50 in FIGURE 5 are two lighted buttons 54 and 56. When the button 54 is depressed, the control module is enabled to control the TGC characteristic. When the button 56 is depressed, the control module is enabled to adjust a lateral gain control (LGC) characteristic, when the ultrasound system is so equipped. Double tapping a button will reset the corresponding gain curve to a nominal TGC or LGC gain characteristic. The color of the button light may be varied to provide additional user information. For instance, a green color may indicate that no adjustment has been made to a nominal TCG curve, and an orange color may indicate that the nominal TCG curve has been altered. Such indicator lights may also be mounted on or correspond to each of the enlarged regions 52 if desired.The number of enlarged regions 52 of the TGC control module is equal to the number of TGC depth zones which may be controlled. In the illustrated example of FIGURES 5 and 6, there are eight enlarged regions 52 for controlling eight depth zones. The touch sensors 60 located on the sides of the enlarged regions may be capacitive sensors or resistive sensors. Those located nearer to the base of the module, when touched, will cause a greater increase or decrease in TGC gain for a particular zone. Touching the sensors near the top of the enlarged regions effect gain adjustment in lesser increments than those caused by touching the bottom, and continual adjustment can be made by holding a finger in contact with a sensor rather than just touching it quickly: the amount of gain adjustment is proportionate to the length of time a sensor is touched. The periodically varying shape of the control module enable a user to sense the controls tactilely by putting a hand on top of the module to feel its raised areas, then sliding the hand down to the enlarged region for the zone of interest. This enables the user to make adjustments to the TGC gain of various zones while keeping his or her eyes focused on the ultrasound image which is to be improved by the results of the adjustments.

Significantly for patient health and safety, the TGC control module 50 requires no slots or other openings in the ultrasound system control panel, unlike the conventional slide pot controls. With its polymeric coating the entire module can be wiped down with a disinfectant during cleaning. From a reliability perspective, the module 50 has no mechanical or moving parts and is thus not subject to the mechanical breakdowns of slide pot devices.

A signal path for processing TGC control signals produced by a TGC control module of the present invention is shown in FIGURE 7. At the left side of the drawing is a cross-sectional view of an enlarged region 52 of a control module showing the touch sensors 60 on each side of the enlarged region. In this example the touch sensors nearest the base of the module will effect a 10% change in TGC gain when touched. The sensors just above them cause a 5% change, and the two uppermost sensors effect 2% and 1% changes, respectively. The sensors on the right side of the enlarged regions cause an increase in TGC gain; those on the left side cause a decrease in gain. When two adjacent sensors are touched at the same time, for instance, the applied gain is a blended value of that controlled by the two sensors. While this example shows discrete sensors, those skilled in the art will appreciate that continuous sensors on each side could be used, with locational sensing of a touched position being used to determine the magnitude of a gain adjustment.

In the discrete sensor example of FIGURE 7, a conductor 63 from each sensor 60 is coupled to an input of a TGC decoder 64 as indicated by the arrow in the drawing. The TGC decoder produces a gain signal appropriate for the touch sensor from which a signal is received. The gain signal may be one of four possible magnitudes (*e.g.*, 10%, 5%, 2%, 1%) and may be either an increase or a decrease in gain. Each new gain signal increment is added to or subtracted from a current gain control signal by a TGC gain accumulator 68. The TGC gain accumulator starts with a gain value provided by a TGC memory 66, which is the gain for the zone provided by the nominal starting TGC gain characteristic. The TGC gain for the zone is then incremented or decremented from the initial gain value.

The gain value of the given depth zone, as increased or decreased by touching its corresponding enlarged region of the control module, is coupled to a TGC segment processor 70. The segment processor also receives the gain values of other depth zones as indicated by input arrows 68' and 68". The TGC segment processor assembles a complete TGC gain characteristic from the gain segment values of the different depth zones, which is sequentially applied to a digital-to-analog converter 72 and used to control the gain applied to the received echo signals by the TGC amplifiers 14. The TGC gain characteristic is also applied to the TGC display processor 38, which processes the TGC characteristic for display as curve 116 on the image display 40.

Other variations will readily occur to those skilled in the art. For instance, the TGC decoder could additionally respond to the touch of a touch sensor by sending a signal to the ultrasound system's audio system, which would respond by producing an audible "click" sound. This would provide audible feedback to the user each time the user increments or decrements a TGC gain value. A higher frequency click would signify an increase in gain and a lower frequency click would signify a decrease in gain. Alternatively, haptic feedback could be delivered to the operator by piezoelectric vibration of the touch sensor.

FIGURE 8 illustrates an example method 800 for producing ultrasound images adjusted for depth-dependent ultrasound attenuation by TGC control. The example method 800 may be implemented on of the above mentioned devices and systems.

The method 800 may adjust a segment 802 of a TGC gain characteristic with a TGC control module 50, wherein the TGC control module comprises a plurality of enlarged regions 52 and at least one touch sensor for each of the plurality of enlarged regions.

The method 800 may apply an adjusted TGC gain 804 to a TGC amplifier with a TGC gain processor in response to input at the at least one touch sensor used to adjust a TGC gain characteristic. Optionally, the method 800 may include displaying an ultrasound image 806 and a curve of the TGC gain characteristic.

It should be noted that an ultrasound system suitable for use in an implementation of the present invention, and in particular the component structure of the ultrasound system of FIGURES 2, 3, and 4, may be implemented in hardware, software or a combination thereof. The various embodiments and/or components of an ultrasound system and its controller, or components and controllers therein, also may be implemented as part of one or more computers or microprocessors. The computer or processor may include a computing device, an input device, a display unit and an interface, for example, for accessing the internet. The computer or processor may include a microprocessor. The microprocessor may be connected to a communication bus, for example, to access a PACS system or the data network for importing training images. The computer or processor may also include a memory. The memory devices such as the TGC memory may include Random Access Memory (RAM) and/or Read Only Memory (ROM). The computer or processor further may include a storage device, which may be a hard disk drive or a removable storage drive such as a floppy disk drive, optical disk drive, solid-state drive, and the like. The storage device may also be other similar techniques for loading computer programs or other instructions into the computer or processor.

As used herein, the term "computer" or "module" or "processor" or "workstation" may include any processor-based or microprocessor-based system including systems using microcontrollers, reduced instruction set computers (RISC), ASICs, logic circuits, and any other circuit or processor capable of executing the functions described herein. The above examples are exemplary only and are thus not intended to limit in any way the definition and/or meaning of these terms.

The computer or processor executes a set of instructions that are stored in one or more storage elements, in order to process input data. The storage elements may also store data or other information as desired or needed. The storage elements may be in the form of an information source or a physical memory element within a processing machine. The set of instructions of an ultrasound system including those controlling the acquisition, processing, and display of ultrasound images as described above may include various commands that instruct a computer or processor as a processing machine to perform specific operations such as the methods and processes of the various embodiments of the invention. Software instructions could be used by the TGC segment processor to assemble a complete TGC characteristic, for instance. The set of instructions may be in the form of a software program. The software may be in various forms such as system software or application software and which may be embodied as a tangible and non-transitory computer readable medium. Numerous ultrasound system functions are typically calculated by or under the direction of software routines. Further, the software may be in the form of a collection of separate programs or modules, or a program module within a larger program or a portion of a program module. The software also may include modular programming in the form of object-oriented programming. The processing of input data by the processing machine may be in response to operator commands, or in response to results of previous processing, or in response to a request made by another processing machine.

## Claims

1. An ultrasonic diagnostic imaging system for producing ultrasound images adjusted for depth-dependent ultrasound attenuation by TGC (time gain compensation) control comprising:
TGC amplifiers (14) located in a receive echo signal path of the imaging system;
a source (80) of a TGC gain characteristic; and
a TGC control module (50) adapted to enable adjustment of the TGC gain characteristic, the TGC control module comprising a plurality of enlarged regions (52), each for adjusting a segment of a TGC gain characteristic and each further comprising a plurality of touch sensors (60) located on opposite sides of an enlarged region; and
a TGC gain processor (16), responsive to the touch sensors and to the TGC gain characteristic, adapted to apply adjusted TGC gain to the TGC amplifiers;
**characterized in that**
the enlarged regions are wider and higher than intermediate areas of the TCG control module;
wherein the TGC control module further comprises an elongated structure with the enlarged regions, the enlarged regions being periodically spaced;
wherein the number of enlarged regions equals the number of TGC depth zones to be controlled, and
wherein the touch sensors on one side of each enlarged region are further adapted to reduce TGC gain and the touch sensors on the opposite side of each enlarged region are further adapted to increase TGC gain.

2. The ultrasonic diagnostic imaging system of Claim 1, wherein the TGC gain processor is further adapted to adjust the TGC gain characteristic in response to the touch sensors; and
further comprising an image display adapted to display an ultrasound image and a curve of the TGC gain characteristic.

3. The ultrasonic diagnostic imaging system of Claim 1, wherein the touch sensors further comprise capacitive touch sensors.

4. The ultrasonic diagnostic imaging system of Claim 1, wherein the touch sensors further comprise resistive touch sensors.

5. The ultrasonic diagnostic imaging system of Claim 1, wherein the receive echo signal path further comprises a beamformer,
wherein the TGC amplifiers are located prior to the beamformer in the receive echo signal path.

6. The ultrasonic diagnostic imaging system of Claim 1, further comprising a light associated with one or more of the enlarged regions,
wherein a color of the light indicates whether a nominal TGC gain characteristic has been changed or not.

7. The ultrasonic diagnostic imaging system of Claim 1, wherein the source of a TGC gain characteristic further comprises a TGC memory.

8. The ultrasonic diagnostic imaging system of Claim 7, wherein the TGC memory is further adapted to provide a nominal initial TGC characteristic at the beginning of an ultrasound procedure.

9. The ultrasonic diagnostic imaging system of Claim 8, wherein the nominal initial TGC characteristic is further adapted to be adjusted by the TGC control module during the ultrasound procedure.

10. The ultrasonic diagnostic imaging system of Claim 1, wherein the plurality of touch sensors are further adapted to provide different amounts of TGC gain adjustment.

11. The ultrasonic diagnostic imaging system of Claim 1, wherein touch sensors lower on an enlarged region are adapted to provide greater TGC gain adjustment than touch sensors higher on an enlarged region.

12. A method for producing ultrasound images adjusted for depth-dependent ultrasound attenuation by TGC (time gain compensation) control comprising:
adjusting a segment of a TGC gain characteristic with a TGC control module (50), wherein the TGC control module comprises a plurality of enlarged regions (52) and at least one touch sensor for each of the plurality of enlarged regions; and
apply an adjusted TGC gain to a TGC amplifier with a TGC gain processor in response to input at the at least one touch sensor used to adjust a TGC gain characteristic;
**characterized in that**
the enlarged regions are wider and higher than intermediate areas of the TCG control module;
wherein the TGC control module further comprises an elongated structure with the enlarged regions, the enlarged regions being periodically spaced;
wherein the number of enlarged regions equals the number of TGC depth zones to be controlled, and
wherein the touch sensors on one side of each enlarged region are further adapted to reduce TGC gain and the touch sensors on the opposite side of each enlarged region are further adapted to increase TGC gain.

## Patentansprüche

1. Diagnostisches Ultraschallbildgebungssystem zum Erzeugen von Ultraschallbildern, die durch TGC- (Zeitverstärkungsausgleich) Steuerung an tiefenabhängige Ultraschalldämpfung angepasst sind, umfassend:
TGC-Verstärker (14), die sich in einem Empfangsechosignalweg des Bildgebungssystems befinden;
eine Quelle (80) einer TGC-Verstärkungscharakteristik; und
ein TGC-Steuerungsmodul (50), das dazu ausgelegt ist, eine Anpassung der TGC-Verstärkungscharakteristik zu ermöglichen, wobei das TGC-Steuerungsmodul eine Vielzahl von vergrößerten Bereichen (52) umfasst, von denen jeder zum Anpassen eines Segments einer TGC-Verstärkungscharakteristik vorgesehen ist und von denen jeder weiter eine Vielzahl von Berührungssensoren (60) umfasst, die sich auf gegenüberliegenden Seiten eines vergrößerten Bereichs befinden; und
einen TGC-Verstärkungsprozessor (16), der auf die Berührungssensoren und die TGC-Verstärkungscharakteristik reagiert und dazu ausgelegt ist, eine angepasste TGC-Verstärkung auf die TGC-Verstärker anzuwenden;
**dadurch gekennzeichnet, dass**
die vergrößerten Bereiche breiter und höher sind als Zwischenbereiche des TGC-Steuerungsmoduls;
wobei das TGC-Steuerungsmodul weiter eine längliche Struktur mit den vergrößerten Bereichen umfasst, wobei die vergrößerten Bereiche periodisch beabstandet sind;
wobei die Anzahl der vergrößerten Bereiche der Anzahl der zu steuernden TGC-Tiefenzonen entspricht, und
wobei die Berührungssensoren auf einer Seite jedes vergrößerten Bereichs weiter dazu ausgelegt sind, die TGC-Verstärkung zu verringern, und die Berührungssensoren auf der gegenüberliegenden Seite jedes vergrößerten Bereichs weiter dazu ausgelegt sind, die TGC-Verstärkung zu erhöhen.

2. Diagnostisches Ultraschallbildgebungssystem nach Anspruch 1, wobei der TGC-Verstärkungsprozessor weiter dazu ausgelegt ist, die TGC-Verstärkungscharakteristik als Reaktion auf die Berührungssensoren anzupassen; und
weiter umfassend eine Bildanzeige, die dazu ausgelegt ist, ein Ultraschallbild und eine Kurve der TGC-Verstärkungscharakteristik anzuzeigen.

3. Diagnostisches Ultraschallbildgebungssystem nach Anspruch 1, wobei die Berührungssensoren weiter kapazitive Berührungssensoren umfassen.

4. Diagnostisches Ultraschallbildgebungssystem nach Anspruch 1, wobei die Berührungssensoren weiter resistive Berührungssensoren umfassen.

5. Diagnostisches Ultraschallbildgebungssystem nach Anspruch 1, wobei der Empfangsechosignalweg weiter einen Strahlformer umfasst,
wobei die TGC-Verstärker sich im Empfangsechosignalweg vor dem Strahlformer befinden.

6. Diagnostisches Ultraschallbildgebungssystem nach Anspruch 1, weiter umfassend ein Licht, das einem oder mehreren der vergrößerten Bereiche zugeordnet ist,
wobei eine Farbe des Lichts angibt, ob eine nominale TGC-Verstärkungscharakteristik geändert wurde oder nicht.

7. Diagnostisches Ultraschallbildgebungssystem nach Anspruch 1, wobei die Quelle einer TGC-Verstärkungscharakteristik weiter einen TGC-Speicher umfasst.

8. Diagnostisches Ultraschallbildgebungssystem nach Anspruch 7, wobei der TGC-Speicher weiter dazu ausgelegt ist, zu Beginn einer Ultraschallprozedur eine nominale anfängliche TGC-Charakteristik bereitzustellen.

9. Diagnostisches Ultraschallbildgebungssystem nach Anspruch 8, wobei die nominale anfängliche TGC-Charakteristik weiter dazu ausgelegt ist, während der Ultraschallprozedur durch das TGC-Steuerungsmodul angepasst zu werden.

10. Diagnostisches Ultraschallbildgebungssystem nach Anspruch 1, wobei die Vielzahl von Berührungssensoren weiter dazu ausgelegt ist, unterschiedliche Mengen an TGC-Verstärkungsanpassung bereitzustellen.

11. Diagnostisches Ultraschallbildgebungssystem nach Anspruch 1, wobei Berührungssensoren, die sich weiter unten auf einem vergrößerten Bereich befinden, dazu ausgelegt sind, eine größere TGC-Verstärkungsanpassung bereitzustellen als Berührungssensoren, die sich weiter oben auf einem vergrößerten Bereich befinden.

12. Erzeugungsverfahren von Ultraschallbildern, die durch TGC-(Zeitverstärkungsausgleich) Steuerung an tiefenabhängige Ultraschalldämpfung angepasst sind, umfassend:
Anpassen eines Segments einer TGC-Verstärkungscharakteristik mit einem TGC-Steuerungsmodul (50), wobei das TGC-Steuerungsmodul eine Vielzahl von vergrößerten Bereichen (52) und mindestens einen Berührungssensor für jeden der Vielzahl von vergrößerten Bereichen umfasst; und
Anwenden einer angepassten TGC-Verstärkung auf einen TGC-Verstärker mit einem TGC-Verstärkungsprozessor als Reaktion auf eine Eingabe an dem mindestens einen Berührungssensor, der zum Anpassen einer TGC-Verstärkungscharakteristik verwendet wird;
**dadurch gekennzeichnet, dass**
die vergrößerten Bereiche breiter und höher sind als Zwischenbereiche des TGC-Steuerungsmoduls;
wobei das TGC-Steuerungsmodul weiter eine längliche Struktur mit den vergrößerten Bereichen umfasst, wobei die vergrößerten Bereiche periodisch beabstandet sind;
wobei die Anzahl der vergrößerten Bereiche der Anzahl der zu steuernden TGC-Tiefenzonen entspricht, und
wobei die Berührungssensoren auf einer Seite jedes vergrößerten Bereichs weiter dazu ausgelegt sind, die TGC-Verstärkung zu verringern, und die Berührungssensoren auf der gegenüberliegenden Seite jedes vergrößerten Bereichs weiter dazu ausgelegt sind, die TGC-Verstärkung zu erhöhen.

## Revendications

1. Système d'imagerie diagnostique ultrasonore pour la production d'images ultrasonores ajustées à l'atténuation ultrasonore en fonction de la profondeur par commande TGC (time gain compensation) comprenant :
des amplificateurs TGC (14) situés dans un chemin de signal d'écho de réception du système d'imagerie ;
une source (80) d'une caractéristique de gain TGC ; et
un module de commande TGC (50) adapté pour permettre l'ajustement de la caractéristique de gain TGC, le module de commande TGC comprenant une pluralité de régions agrandies (52), chacune permettant d'ajuster un segment d'une caractéristique de gain TGC et comprenant en outre une pluralité de capteurs tactiles (60) situés sur des côtés opposés d'une région agrandie ; et
un processeur de gain TGC (16), sensible aux capteurs tactiles et à la caractéristique de gain TGC, adapté pour appliquer un gain TGC ajusté aux amplificateurs TGC ;
**caractérisé en ce que**
les régions agrandies sont plus larges et plus hautes que des zones intermédiaires du module de commande TCG ;
dans lequel le module de commande TGC comprend en outre une structure allongée avec des régions agrandies, les régions agrandies étant espacées périodiquement ;
dans lequel le nombre de régions agrandies est égal au nombre de zones de profondeur TGC à commander, et
dans lequel les capteurs tactiles d'un côté de chaque région agrandie sont en outre adaptés pour réduire le gain TGC et les capteurs tactiles du côté opposé de chaque région agrandie sont en outre adaptés pour augmenter le gain TGC.

2. Système d'imagerie diagnostique ultrasonore selon la revendication 1, dans lequel le processeur de gain TGC est en outre adapté pour ajuster la caractéristique de gain TGC en réponse aux capteurs tactiles ; et
comprenant en outre un écran d'affichage adapté à l'affichage d'une image ultrasonore et d'une courbe de la caractéristique de gain TGC.

3. Système d'imagerie diagnostique ultrasonore selon la revendication 1, dans lequel les capteurs tactiles comprennent en outre des capteurs tactiles capacitifs.

4. Système d'imagerie diagnostique ultrasonore selon la revendication 1, dans lequel les capteurs tactiles comprennent en outre des capteurs tactiles résistifs.

5. Système d'imagerie diagnostique ultrasonore de la revendication 1, dans lequel le chemin de signal d'écho de réception comprend en outre un formateur de faisceau,
dans lequel les amplificateurs TGC sont situés en amont du formateur de faisceau dans le chemin du signal d'écho de réception.

6. Système d'imagerie diagnostique ultrasonore selon la revendication 1, comprenant en outre une lumière associée à une ou plusieurs des régions agrandies,
dans lequel la couleur de la lumière indique si une caractéristique de gain TGC nominale a été modifiée ou non.

7. Système d'imagerie diagnostique ultrasonore selon la revendication 1, dans lequel la source d'une caractéristique de gain TGC comprend en outre une mémoire TGC.

8. Système d'imagerie diagnostique ultrasonore selon la revendication 7, dans lequel la mémoire TGC est en outre adaptée pour fournir une caractéristique TGC initiale nominale au début d'une procédure ultrasonore.

9. Système d'imagerie diagnostique ultrasonore selon la revendication 8, dans lequel la caractéristique TGC initiale nominale est en outre adaptée pour être ajustée par le module de commande TGC pendant la procédure ultrasonore.

10. Système d'imagerie diagnostique ultrasonore selon la revendication 1, dans lequel la pluralité de capteurs tactiles est en outre adaptés pour fournir différentes ampleurs d'ajustement de gain TGC.

11. Système d'imagerie diagnostique ultrasonore selon la revendication 1, dans lequel des capteurs tactiles situés plus bas sur une région agrandie sont adaptés pour fournir un ajustement de gain TGC plus important que des capteurs tactiles situés plus haut sur une région agrandie.

12. Procédé de production d'images ultrasonores ajustées à l'atténuation ultrasonore en fonction de la profondeur par commande TGC (time gain compensation) comprenant :
l'ajustement d'un segment d'une caractéristique de gain TGC avec un module de commande TGC (50), dans lequel module de commande TGC comprend une pluralité de régions agrandies (52) et au moins un capteur tactile pour chacune de la pluralité de régions agrandies ; et
l'application d'un gain TGC ajusté à un amplificateur TGC avec un processeur de gain TGC en réponse à une entrée au niveau de l'au moins un capteur tactile utilisé pour ajuster une caractéristique de gain TGC ;
**caractérisé en ce que**
les régions agrandies sont plus larges et plus hautes que des zones intermédiaires du module de commande TCG ;
dans lequel le module de commande TGC comprend en outre une structure allongée avec des régions agrandies, les régions agrandies étant espacées périodiquement ;
dans lequel le nombre de régions agrandies est égal au nombre de zones de profondeur TGC à commander, et
dans lequel les capteurs tactiles d'un côté de chaque région agrandie sont en outre adaptés pour réduire le gain TGC et les capteurs tactiles du côté opposé de chaque région agrandie sont en outre adaptés pour augmenter le gain TGC.
